# EUROPEAN PATENT APPLICATION

(11) **EP 1 076 052 A2**
(43) Date of publication of application: **14.02.2001**
(21) Application number: 00306903.6
(22) Date of filing: 11.08.2000
(51) Int. Cl.: C07C 227/44, C07C 229/24

(54) **L-Asparate solution and a storage and/or transportation method thereof**

(30) Priority: 12.08.1999 JP 22868899; 12.08.1999 JP 22868999
(71) Applicant: Nippon Shokubai Co., Ltd., Osaka-shi, Osaka 541-0043 (JP)
(72) Inventor: Mukouyama, Masaharu, Tsukuba-gun, Ibaraki 300-2301 (JP); Satonaka, Satomi, Sapporo-shi, Hokkaido 065-0022 (JP)
(74) Representative: Maschio, Antonio

(57) **Abstract**

The present invention relates to ammonium L-aspartate solution wherein the concentration and temperature for ammonium L-aspartate are maintained in the region bounded by A, B, C, D, E, F, G, H, I and J as shown in Fig.1. The region above for the concentration and temperature is preferably bounded by B, C, D, E, F, G, H and I as shown in Fig. 1, more preferably, bounded by C, D, E, F, G and H as shown in Fig.1.

## Description

### Field of the Invention

The present invention relates to aspartic acid salt solution containing L-aspartate and a method for storing and transporting the aspartic acid salt solution.

### Background of the Invention

The L-aspartate is produced by reacting fumaric acid with ammonia through the activity of aspartase. Such a L-aspartate is ammonium L-aspartate directly generated from the reaction. Such L-aspartates include sodium L-aspartate, which is synthesized by adding sodium hydroxide in an ammonium L-aspartate solution followed by salt exchange, potassium L-aspartate and magnesium L-aspartate, which are similarly synthesized by salt exchange reaction. Methods for preparing these salt solutions are disclosed in Japanese Patent Laid-Open Publication Nos. 58-170480, and 9-202758.

In the process disclosed in Japanese Patent Laid-Open Publication No. 9-202758, ammonium L-aspartate solution, which is produced by reacting fumaric acid with ammonia, and sodium hydroxide together are poured into a distillation column from the upper portion, by which free ammonia and some water are obtained from the upper portion of the distillation column in addition to sodium L-aspartate synthesized by salt exchange from the lower portion of the distillation column.

However, there exist some problems in the process described above when the solution is stored and transported in large quantities. Since L-aspartate solution obtained in such a process is low in its concentration, usually ranging from 20 to 30 wt %, the process requires a storage bath large enough to handle solution containing water in far larger amount than that of L-aspartate, which leads to a high cost in transportation.

No such problem occurs when L-aspartate solution is neither stored nor transported, but used as a material at its intact concentration for subsequent reaction. Such a case is that L-aspartate is subjected to reaction with acid chloride of fatty acid to produce a surfactant.

However, when L-aspartate solution is not used immediately after its production, there is a need to maintain, or store or transport the solution together with water in large quantities when the next process or reaction, such as reaction to produce a surfactant as described above, is performed in another place.

To solve the above problems, normally L-aspartate isolated as free acid crystal is stored and transported. Then at a place for the next process, alkaline hydroxide metal and alkaline hydroxide earth metal are added to the L-aspartate solution to prepare corresponding salt.

This process, however, requires complex steps and facilities for precipitating and filtering crystal in order to separate L-aspartic acid as free acid crystal, leading to a high cost. The step of dissolving L-aspartic acid, which have once been separated from solution, in water by neutralizing the aspartic acid is very ineffective.

To settle these problems, development of a process to prepare stable L-asparic acid salt solution, which is not altered even at high concentration such that it is separated as crystal, has been expected.

### Summary of the Invention

The object of the present invention is to provide stable L-aspartate solution, which can be stored for long term. Another object of this invention is to provide a method for storing and/or transporting L-aspartate. That is, the object of the invention is to provide a method for storing and/or transporting L-aspartate in the form of ammonium L-aspartate solution, monosodium L-aspartate solution, 1.5-sodium L-aspartate solution, 0.5-calcium L-aspartate solution or 2-sodium L-aspartate solution, which are not altered such that separated as crystal, for a long term, at least more than one month.

As a result of diligent research to solve the problems above, the present inventors have completed the present invention by finding that conditions for L-aspartate to be precipitated as salt differ in the type of L-aspartate solution, and its dissolution concentration (molar ratio) and that surprisingly, at a specific concentration and within a specific temperature range, no crystal is precipitated.

The present invention relates to L-aspartic acid ammonium, sodium, potassium, magnesium or calcium L-aspartate, whose molar ratio is normally 1-fold mole for ammonium, 0.8 to 2.2-fold mole for sodium and potassium, and 0.4 to 0.6-fold mole for calcium; and a method for storing and/or transporting L-aspartate solution whose concentration is 30 wt % or more when it was estimated in salt.

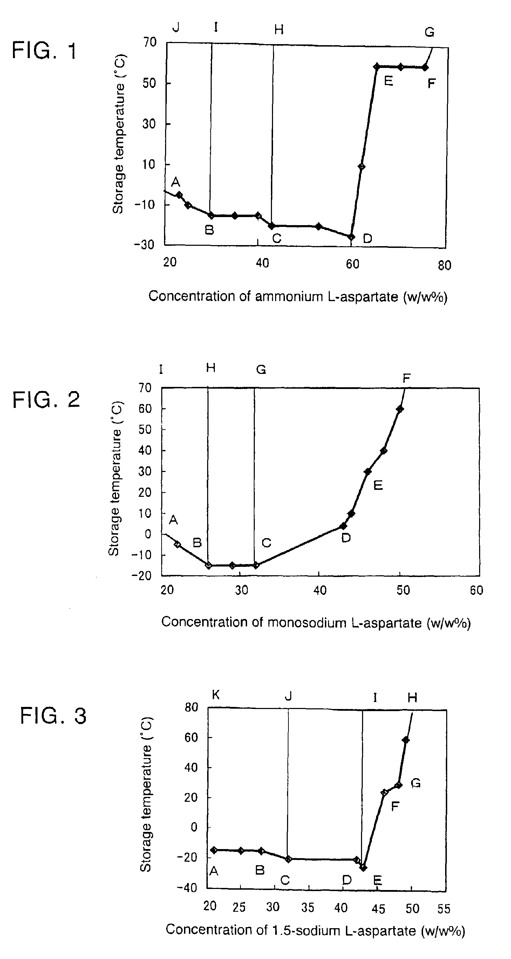

That is, the present invention relates to ammonium L-aspartate solution whose concentration and temperature are maintained within the region bounded by A, B, C, D, E, F, G, H, I and J as shown in Fig. 1. This region for the concentration and temperature is preferably bounded by B, C, D, E, F, G, H and I, more preferably, bounded by C, D, E, F, G and H.

Further the present invention relates to a method for storing and/or transporting ammonium L-aspartate comprising the step of storing and/or transporting ammonium L-aspartate in ammonium L-aspartate solution with concentration and temperature maintained within the region as described above.

Furthermore the present invention relates to monosodium L-aspartate solution, wherein the concentration and temperature of monosodium L-aspartate are maintained within a region bounded by A, B, C, D, E, F, G, H and I as shown in Fig. 2. This range for the concentration and temperature is preferably bounded by B, C, D, E, F, G, and H as shown in Fig. 2, more preferably, bounded by C, D, E, F, and G as shown in Fig. 2.

Further the present invention relates to a method for storing and/or transporting Monosodium L-aspartate comprising the step of storing and/or transporting Monosodium L-aspartate in Monosodium L-aspartate solution with concentration and temperature maintained within the region as described above.

The present invention also relate to 1.5-sodium L-aspartate solution, wherein the concentration and temperature of 1.5-sodium L-aspartate are maintained within a region bounded by A, B, C, D, E, F, G, H, I, J and K as shown in Fig. 3. This range for the concentration and temperature is preferably bounded by C, D, E, F, G, H, I and J as shown in Fig. 3, more preferably, bounded by E, F, G, H and I as shown in Fig. 3.

Further the present invention relates to a method for storing and/or transporting 1.5-sodium L-aspartate comprising the step of storing and/or transporting 1.5-sodium L-aspartate in 1.5-sodium L-aspartate solution with concentration and temperature maintained within the region as described above.

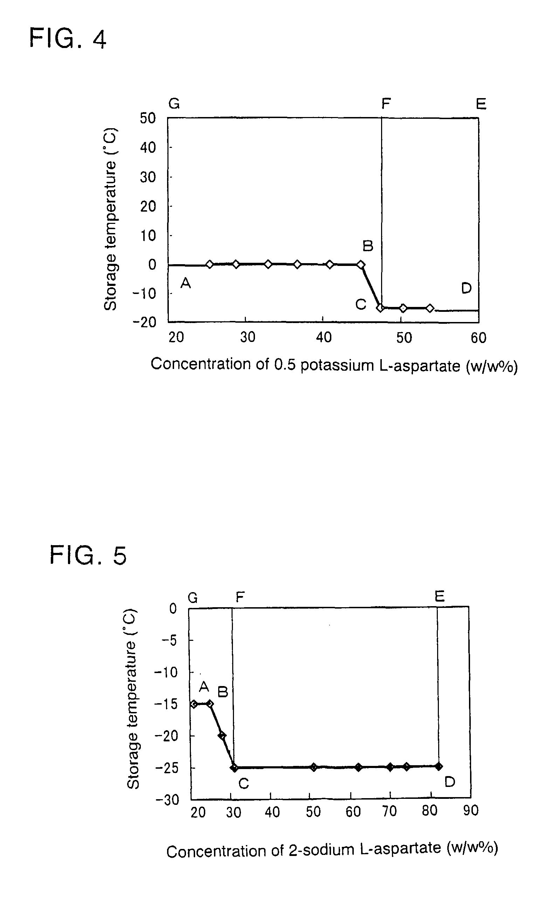

The present invention also relates to 0.5-calcium L-aspartate solution, wherein the concentration and temperature of 0.5-calcium L-aspartate are maintained within a region bounded by A, B, C, D, E, F, and G as shown in Fig. 4. This range for the concentration and temperature is preferably bounded by C, D, E, and F as shown in Fig. 4.

Further the present invention relates to a method for storing and/or transporting 0.5-calcium L-aspartate comprising the step of storing and/or transporting 0.5-calcium L-aspartate in 0.5-calcium L-aspartate solution with concentration and temperature maintained within the region as described above.

The present invention relates to 2-sodium L-aspartate solution, whose molar ratio is normally 1.8 to 2.2-fold mole and whose concentration is 30 wt % or more when it is estimated in salt; and a method for storing and/or transporting the 2-sodium L-aspartate solution.

The present invention also relates to 2-sodium L-aspartate solution, wherein the concentration and temperature of 2-sodium L-aspartate are maintained within a region bounded by A, B, C, D, E, F, and G as shown in Fig. 5. This range for the concentration and temperature is preferably bounded by C, D, E, and F as shown in Fig. 5.

Further the present invention relates to a method for storing and/or transporting 2-sodium L-aspartate comprising the step of storing and/or transporting 2-sodium L-aspartate in 2-sodium L-aspartate solution with concentration and temperature maintained within the region as described above.

This specification includes part or all of the contents as disclosed in the specification and/or drawings of Japanese Patent Application Nos. 11-228688, and 11-228689, each of which is a priority document of the present application.

### Brief Description of the Drawings

Figure 1 shows dissolution stability of ammonium L-aspartate in water.

Figure 2 shows dissolution stability of monosodium L-aspartate in water.

Figure 3 shows dissolution stability of 1.5-sodium L-aspartate in water.

Figure 4 shows dissolution stability of 0.5 potassium L-aspartate in water.

Figure 5 shows dissolution stability of 2-sodium L-aspartate in water.

### Detailed Description of the Preferred Embodiments

The present invention will now be described in detail.

The L-aspartate used in this invention includes ammonium L-aspartate, sodium L-aspartate and potassium L-aspartate as alkaline metal salt, and calcium L-aspartate as alkaline earth metal salt. Alkaline metal salt and alkaline earth metal salt are preferable among them.

These L-aspartates are produced by reacting fumaric acid with ammonia through the activity of aspartase. Typical L-aspartate is ammonium L-aspartate, which is directly generated by such a reaction. The L-aspartate includes sodium L-aspartate, which is synthesized by adding sodium hydroxide to the L-aspartic acid ammonium followed by salt exchange, potassium L-aspartate, magnesium L-aspartate, and calcium L-aspartate, which are also synthesized through salt exchange reaction.

The above-mentioned aspartase can be produced by culturing *Escherichia coli* ATCC 11303 strain. In addition to this aspartase purified from the microorganism, aspartase contained in culture fluid of this *E.coli* ATCC 11303 strain or its processed matter can also be used in this invention.

The concentrated solution of L-aspartate solution can be produced by subjecting L-aspartate solution to an evaporator or by dissolving L-aspartate in water.

The method for preparing ammonium L-aspartate, monosodium L-aspartate, 1.5-sodium L-aspartate, potassium L-aspartate and 2-sodium L-aspartate is described as follows.

First, ammonium L-aspartate is prepared as described below. L-aspartic acid is produced by reacting fumaric acid and ammonia in the presence of an enzyme, aspartase as described above.

At this time, since diammonium fumarate has a solubility of 250g/L at normal temperature, ammonium L-aspartate is about a 21% solution. Normally, the ratio of the presence of L-aspartic acid and ammonia in the ammonium L-aspartate solution, which is produced through enzymatic reaction, is 1:1 to 1:4. Ammonia forms salt by reacting with the first carboxyl group, but forms no salt with the second carboxyl group among two carboxyl groups contained in aspartic acid, because of its weak basicity of ammonia. Therefore, ammonium L-aspartate is normally present as monoammonium.

Such monoammonium L-aspartate solution is heated at 50 °C to 130 °C to evaporate water, thereby obtaining the concentrated monoammonium L-aspartate solution. The solution can be concentrated by a known method wherein the solution is concentrated using a rotary evaporator under reduced pressure at 50 °C to 95 °C; a method wherein the solution is heated and evaporated under normal pressure below 160 °C; or a method wherein water and excess ammonia are removed from the upper portion of a distillation column containing the solution introduced therein, and concentrated solution is collected from the bottom.

Next, monosodium L-aspartate is prepared as follows. Sodium hydroxide with one-fold mole for ammonia, which is present in the reaction solution, is added to the monoammonium L-aspartate solution produced in the presence of an aspartase enzyme, and then heated. Ammonia is easily liberated by this heat since ammnoia has a weaker basicity than that of sodium hydroxide.

This solution, to which sodium hydroxide is added, is heated at 50 °C to 130 °C to evaporate water so that the concentrated monosodium L-aspartate solution is synthesized.

1.5-sodium L-aspartate is prepared as follows. Sodium hydroxide with 1.5-fold mole for ammonia, which is present in the reaction solution, is added to the monoammonium L-aspartate solution produced in the presence of an aspartase enzyme, and then heated. Subsequently the solution is concentrated similarly, so that 1.5-sodium L-aspartate solution is synthesized.

0.5-calcium L-aspartate is prepared as follows. Calcium hydroxide with 0.5-fold mole for ammonia, which is present in the reaction solution, is added to the monoammonium L-aspartate solution produced in the presence of an aspartase enzyme, and then heated. Subsequently the solution is concentrated similarly, so that 0.5-calcium L-aspartate solution is synthesized.

2-sodium L-aspartate is prepared as follows. Sodium hydroxide with 2-fold mole for ammonia, which is present in the reaction solution, is added to the monoammonium L-aspartate solution produced in the presence of an aspartase enzyme, and then heated. Subsequently the solution is concentrated similarly, so that 2-sodium L-aspartate solution is synthesized.

### Examples

The present invention is further described in the following examples. These examples are provided for illustrative purposes only, and are not intended to limit the scope of the invention.

### Example 1

60 L of a medium at pH 6.3 with a composition per litter of distilled water consisted of 10 g of fumaric acid, 5g of ammonium sulfate, lg of monopotassium phosphate, 3g of dipotassium phosphate, 0.5g of magnesium sulfate heptahydrate, 5.5g of NaOH and 20g of yeast extract, was placed in a 90L jar fermenter. *Escherichia coli* ATCC11303 strain, which has been pre-cultured in 1L of a medium with the above composition, was inoculated in the fermenter and cultured at 37 °C under aeration and agitation. After 12 hours the culture was completed, and butter-like cells were collected by centrifugation.

18g of PAS-880 (Nitto Boseki Co., Ltd) adjusted to around pH 7 with alkaline and 27g of deionized water were mixed well for uniform dispersion of 40g of the above cells. 300 ml of ion exchange resin (Amberlight IRA-96SB, C1 type, manufactured by Organo Corp., 0.5mm of average grain diameter) and previously obtained cell suspension were added in a 6L Kjeldahl shortneck flask. The mixture in this flask was dried under reduced pressure for 6 hours while rotating at 30 °C so that the cells were bound to the surface of the ion exchange resin, thereby obtaining an immobilized biocatalyst. The water content of this immobilized biocatalyst measured with a Carl-Fisher moisture meter was 6 wt%.

The immobilized biocatalyst prepared as described above was dipped in 20% ammonium fumarate solution at pH 8.5 at 4 °C overnight. Then 25ml of the resulting product was packed in a column with a jacket. Hot water at 30 °C was allowed to circulate in the jacket and the temperature of a reactor was set at 30 °C.

Substrate solution in a bottle with a cap had a composition per litter consisted of 200 g of fumaric acid, 200g of 25% aqueous ammonia, and 0.25g of magnesium sulfate heptahydrate, and adjusted to pH 8.3 with ammonia. The substrate solution was introduced into the column at a rate of 25ml per hour through a Teflon tube so that reaction took place continuously.

Analysis of the reaction solution after 6 hours of the reaction has started revealed that L-aspartic acid was generated as reaction products in equimolar quantity with the fumaric acid consumed. The reaction conversion rate was 99.7%. One month and four months after the reaction has started, the conversion rate was kept at 99.7%.

This 1L of 23% ammonium L-aspartate solution (specific gravity 1.11) was added in a 2L Kjeldahl shortneck flask, and concentrated under reduced pressure with an evaporator while dipping in a thermostat at 60 °C. About 1 hour after this manipulation, 599g of ammonium L-aspartate solution remained in the Kjeldahl shortneck flask. No precipitated crystal was seen during and after the manipulation. The concentration of this solution was 42.65 w/w % when it was estimated in salts. That is, the solution could be concentrated about 1.9 fold.

No precipitated crystal was observed even when this concentrated solution was allowed to stand at 20 °C for 3 days. Moreover, when refrigerated at 4 °C for 3 days, no precipitated crystal was observed. Subsequent refrigeration at -5 °C for 3 days of this solution also resulted in no precipitated crystal but the presence of a few insoluble matters.

### Example 2

1L of 23 % ammonium L-aspartate solution obtained in the manner as described in Example 1 was added in a 2L Kjeldahl shortneck flask, and concentrated under reduced pressure with an evaporator while dipping in a thermostat at 60 °C. About 80 minutes after this manipulation, 483g of ammonium L-aspartate solution remained in the Kjeldahl shortneck flask. No precipitated crystal was seen during and after the manipulation. The concentration of this solution was 52.87 W/W % when it was estimated in salts. That is, the solution could be concentrated about 2.3 fold.

No precipitated crystal was observed even when this concentrated solution was allowed to stand at 20 °C for 3 days. Moreover, when refrigerated at 4 °C for 3 days, no precipitated crystal was observed. Subsequent refrigeration of this solution at-5 °C for 3 days also resulted in no precipitated crystal but the presence of a few insoluble matters.

### Example 3

1L of 23% ammonium L-aspartate solution obtained in the manner as described in Example 1 was added in a 2L Kjeldahl shortneck flask, and concentrated under reduced pressure with an evaporator while dipping in a thermostat at 60 °C. About 90 minutes after this manipulation, 423g of ammonium L-aspartate solution remained in the Kjeldahl shortneck flask. No precipitated crystal was seen during and after the manipulation. The concentration of this solution was 60.32 W/W % when it was estimated in salts. That is, the solution could be concentrated about 2.6 fold. The viscosity of this concentrated solution measured were 0.031 Pas·s at 40 °C, 0.053 Pas·s at 25 °C, and 0.149 Pas·s at 4 °C.

No precipitated crystal was observed even when this concentrated solution was allowed to stand at 20 °C for 3 days. Moreover, when refrigerated at 4 °C for 3 days, no precipitated crystal was observed. Subsequent refrigeration of this solution at-5 °C for 3 days also resulted in no precipitated crystal but the presence of a few insoluble matters.

### Example 4

1L of 23% ammonium L-aspartate solution obtained in the manner as described in Example 1 was added in a 2L Kjeldahl shortneck flask, and concentrated under reduced pressure with an evaporator while dipping in a thermostat at 60 °C. About 90 minutes after this manipulation, 415g of ammonium L-aspartate solution remained in the Kjeldahl shortneck flask. No precipitated crystal was seen during and after the manipulation. The concentration of this solution was 61.59 W/W % when it was estimated in salts. That is, the solution could be concentrated about 2.7 fold. The specific gravity of this concentrated solution was 1.29.

No precipitated crystal was observed even when this concentrated solution was allowed to stand at 20 °C for 3 days. When refrigerated at 4 °C, however, precipitated crystal was observed.

### Example 5

1L of 23% ammonium L-aspartate solution obtained in the manner as described in Example 1 was added in a 2L Kjeldahl shortneck flask, and concentrated under reduced pressure with an evaporator while dipping in a thermostat at 60 °C. About 100 minutes after this manipulation, 390g of ammonium L-aspartate solution remained in the Kjeldahl shortneck flask. No precipitated crystal was seen during and after the manipulation. The concentration of this solution was 65.49 W/W % when it was estimated in salts. That is, the solution could be concentrated about 2.9 fold. When this solution was allowed to stand at 20 °C, precipitated crystal was observed.

### Example 6

1L of 23% ammonium L-aspartate solution obtained in the manner as described in Example 1 was added in a 2L Kjeldahl shortneck flask, and concentrated under reduced pressure with an evaporator while dipping in a thermostat at 60 °C. About 100 minutes after this manipulation, 362g of ammonium L-aspartate solution remained in the Kjeldahl shortneck flask. The concentration of this solution was 70 W/W % when it was estimated in salts. No precipitated crystal was observed during the manipulation at 60 °C. When the solution was transferred to another container, however, precipitated crystal was observed.

### Example 7

1L of 23% ammonium L-aspartate solution obtained in the manner as described in Example 1 was added in a 2L Kjeldahl shortneck flask, and concentrated under reduced pressure with an evaporator while dipping in a thermostat at 60 °C. The behavior (dissolution, crystallization, or freezing) of ammonium L-aspartate solution at each temperature and concentration was shown in Table 1.

**Table 1**

| Behavior of ammonium L-aspartate solution at each temperature and concentration | | | | | | | | | | | | |
|---|---|---|---|---|---|---|---|---|---|---|---|---|
| Storage temp. (°C) | Concentration of ammonium L-aspartate (W/W %) | | | | | | | | | | | |
| | 23 | 25 | 30 | 35 | 40 | 43 | 53 | 60 | 62 | 65 | 70 | 75 |
| 60 | ○ | ○ | ○ | ○ | ○ | ○ | ○ | ○ | ○ | ○ | ○ | ○ |
| 40 | ○ | ○ | ○ | ○ | ○ | ○ | ○ | ○ | ○ | precipitated | precipitated | precipitated |
| 30 | ○ | ○ | ○ | ○ | ○ | ○ | ○ | ○ | ○ | precipitated | precipitated | precipitated |
| 25 | ○ | ○ | ○ | ○ | ○ | ○ | ○ | ○ | ○ | precipitated | precipitated | precipitated |
| 10 | ○ | ○ | ○ | ○ | ○ | ○ | ○ | ○ | ○ | precipitated | precipitated | precipitated |
| 4 | ○ | ○ | ○ | ○ | ○ | ○ | ○ | ○ | precipitated | precipitated | precipitated | precipitated |
| 0 | ○ | ○ | ○ | ○ | ○ | ○ | ○ | ○ | precipitated | precipitated | precipitated | precipitated |
| -5 | ○ | ○ | ○ | ○ | ○ | ○ | ○ | ○ | precipitated | precipitated | precipitated | precipitated |
| -10 | frozen | ○ | ○ | ○ | ○ | ○ | ○ | ○ | precipitated | precipitated | precipitated | precipitated |
| -15 | frozen | frozen | ○ | ○ | ○ | ○ | ○ | ○ | precipitated | precipitated | precipitated | precipitated |
| -20 | frozen | frozen | frozen | frozen | frozen | ○ | ○ | ○ | precipitated | precipitated | precipitated | precipitated |
| -25 | frozen | frozen | frozen | frozen | frozen | frozen | *frozen | ○ | precipitated | precipitated | precipitated | precipitated |
| ○ : dissolved, *frozen: partially frozen | | | | | | | | | | | | |

### Example 8

60 L of a medium at pH 6.3 with a composition per litter of distilled water consisted of 10 g of fumaric acid, 5g of ammonium sulfate, 1g of monopotassium phosphate, 3g of dipotassium phosphate, 0.5g of magnesium sulfate heptahydrate, 5.5g of NaOH and 20g of yeast extract, was placed in a 90L jar fermenter. *Escherichia coli* ATCC11303 strain, which has been pre-cultured in 1L of a medium with the above composition, was inoculated in the fermenter and cultured at 37 °C under aeration and agitation. After 12 hours the culture was completed, and butter-like cells were collected by centrifugation.

18g of PAS-880 (Nitto Boseki Co., Ltd) adjusted to around pH 7 with alkaline and 27g of deionized water were mixed well for uniform dispersion of 40g of the above cells. 300 ml of ion exchange resin (Amberlight IRA-96SB, Cl type, manufactured by Organo Corp., 0.5mm of average grain diameter) and previously obtained cell suspension were added in a 6L Kjeldahl shortneck flask. This flask was dried under reduced pressure for 6 hours while rotating at 30 °C so that the cells were bound to the surface of the ion exchange resin, thereby obtaining an immobilized biocatalyst. The water content of this immobilized biocatalyst measured with a Carl-Fisher moisture meter was 6 wt%.

The immobilized biocatalyst prepared as described above was dipped in 20% ammonium fumarate solution at pH 8.5 at 4 °C overnight. Then 25ml of the resulting product was packed in a column with a jacket. Hot water at 30 °C was allowed to circulate in the jacket and the temperature of a reactor was set at 30 °C.

Substrate solution in a bottle with a cap had a composition per litter consisted of 200 g of fumaric acid, 200g of 25% aqueous ammonia, and 0.25g of magnesium sulfate heptahydrate, and adjusted to pH 8.3 with ammonia. The substrate solution was introduced into the column at a rate of 25ml per hour through a Teflon tube so that reaction took place continuously.

Analysis of the reaction solution 6 hours after that the reaction has started revealed that L-aspartic acid was generated as reaction products in equimolar quantity with the fumaric acid consumed. The reaction conversion rate was 99.7%. One month and four months after the reaction has started, the conversion rate was kept at 99.7%.

69g of sodium hydroxide, equimolar with ammonia, was added to this 1L of 23% ammonium L-aspartate solution (specific gravity 1.11). Then the solution (specific gravity 1.17) was added in a 2L Kjeldahl shortneck flask, and concentrated under reduced pressure with an evaporator while dipping in a thermostat at 60 °C. About 1 hour after this manipulation, 657g of monosodium L-aspartate solution remained in the Kjeldahl shortneck flask. No precipitated crystal was seen during and after the manipulation. The concentration of this solution was 40.43 W/W % when it was estimated in salts. That is, the solution could be concentrated about 1.8 fold.

No precipitated crystal was observed even when this concentrated solution was allowed to stand at 20 °C for 3 days. Moreover, when refrigerated at 4 °C for 3 days, no precipitated crystal was observed.

### Example 9

69g of sodium hydroxide, equimolar with ammonia, was added to 1L of 23% ammonium L-aspartate solution obtained in the manner as described in Example 8. Then the solution was added in a 2L Kjeldahl shortneck flask, and concentrated under reduced pressure with an evaporator while dipping in a thermostat at 60 °C. About 80 minutes after this manipulation, 616g of monosodium L-aspartate solution remained in the Kjeldahl shortneck flask. No precipitated crystal was seen during and after the manipulation. The concentration of this solution was 43.06 W/W % when it was estimated in salts. That is, the solution could be concentrated about 1.9 fold.

No precipitated crystal was observed even when this concentrated solution was allowed to stand at 20 °C for 3 days. Moreover, when refrigerated at 4 °C for 3 days, no precipitated crystal was observed.

### Example 10

69g of sodium hydroxide, equimolar with ammonia, was added to 1L of 23% ammonium L-aspartate solution obtained in the manner as described in Example 8. Then the solution was added in a 2L Kjeldahl shortneck flask, and concentrated under reduced pressure with an evaporator while dipping in a thermostat at 60 °C. About 90 minutes after this manipulation, 612g of monosodium L-aspartate solution remained in the Kjeldahl shortneck flask. No precipitated crystal was seen during and after the manipulation. The concentration of this solution was 43.4 W/W % when it was estimated in salts. That is, the solution could be concentrated about 1.9 fold. The viscosity of this concentrated solution measured was 0.007 Pas·s at 40 °C, 0.011 Pas·s at 25 °C, and 0.045 Pas·s at 4 °C.

No precipitated crystal was observed even when this concentrated solution was allowed to stand at 20 °C for 3 days. Moreover, when refrigerated at 4 °C for 3 days, no precipitated crystal was observed.

### Example 11

69g of sodium hydroxide, equimolar with ammonia, was added to 1L of 23% ammonium L-aspartate solution obtained in the manner as described in Example 8. Then the solution was added in a 2L Kjeldahl shortneck flask, and concentrated under reduced pressure with an evaporator while dipping in a thermostat at 60 °C. About 90 minutes after this manipulation, 597g of monosodium L-aspartate solution remained in the Kjeldahl shortneck flask. No precipitated crystal was seen during and after the manipulation. The concentration of this solution was 44.49 W/W % when it was estimated in salts. That is, the solution could be concentrated about 1.92 fold.

No precipitated crystal was observed even when this concentrated solution was allowed to stand at 20 °C for 3 days. When refrigerated at 4 °C, however, precipitated crystal was observed.

### Example 12

69g of sodium hydroxide, equimolar with ammonia, was added to 1L of 23% ammonium L-aspartate solution obtained in the manner as described in Example 8. Then the solution was added in a 2L Kjeldahl shortneck flask, and concentrated under reduced pressure with an evaporator while dipping in a thermostat at 60 °C. About 100 minutes after this manipulation, 569g of monosodium L-aspartate solution remained in the Kjeldahl shortneck flask. No precipitated crystal was seen during and after the manipulation. The concentration of this solution was 46.66 W/W % when it was estimated in salts. That is, the solution could be concentrated about 2 fold. When the solution was allowed to stand at 20 °C, however, precipitated crystal was observed.

### Example 13

69g of sodium hydroxide, equimolar with ammonia, was added to 1L of 23% ammonium L-aspartate solution obtained in the manner as described in Example 8. Then the solution was added in a 2L Kjeldahl shortneck flask, and concentrated under reduced pressure with an evaporator while dipping in a thermostat at 60 °C. About 100 minutes after this manipulation, 553g of monosodium L-aspartate solution remained in the Kjeldahl shortneck flask. The concentration of this solution was 48 W/W % when it was estimated in salts. No precipitated crystal was observed during the manipulation at 60 °C. When the solution was transferred to another container, however, precipitated crystal was observed.

### Example 14

69g of sodium hydroxide, equimolar with ammonia, was added to 1L of 23% ammonium L-aspartate solution obtained in the manner as described in Example 8. Then the solution was added in a 2L Kjeldahl shortneck flask, and concentrated under reduced pressure with an evaporator while dipping in a thermostat at 60 °C. The behavior (dissolution, crystallization, or freezing) of monosodium L-aspartate solution at each temperature and concentration was shown in Table 2.

**Table 2**

| Behavior of monosodium L-aspartate solution at each temperature and concentration | | | | | | | | | |
|---|---|---|---|---|---|---|---|---|---|
| Storage temp. (°C) | Concentration monosodium L-aspartate (w/w%) | | | | | | | | |
| | 22 | 26 | 29 | 32 | 43 | 44 | 46 | 48 | 50 |
| 60 | ○ | ○ | ○ | ○ | ○ | ○ | ○ | ○ | ○ |
| 40 | ○ | ○ | ○ | ○ | ○ | ○ | ○ | ○ | precipitated |
| 30 | ○ | ○ | ○ | ○ | ○ | ○ | ○ | precipitated | precipitated |
| 25 | ○ | ○ | ○ | ○ | ○ | ○ | precipitated | precipitated | precipitated |
| 10 | ○ | ○ | ○ | ○ | ○ | ○ | precipitated | precipitated | precipitated |
| 4 | ○ | ○ | ○ | ○ | ○ | precipitated | precipitated | precipitated | precipitated |
| 0 | ○ | ○ | ○ | ○ | precipitated | precipitated | precipitated | precipitated | precipitated |
| -5 | ○ | ○ | ○ | ○ | precipitated | precipitated | precipitated | precipitated | precipitated |
| -15 | frozen | ○ | ○ | ○ | precipitated | precipitated | precipitated | precipitated | precipitated |
| -20 | frozen | frozen | frozen | frozen | precipitated | precipitated | precipitated | precipitated | precipitated |
| -25 | frozen | frozen | frozen | frozen | precipitated | precipitated | precipitated | precipitated | precipitated |
| ○ : dissolved | | | | | | | | | |

### Example 15

60 L of a medium at pH 6.3 with a composition per litter of distilled water consisted of 10 g of fumaric acid, 5g of ammonium sulfate, 1g of monopotassium phosphate, 3g of dipotassium phosphate, 0.5g of magnesium sulfate heptahydrate, 5.5g of NaOH and 20g of yeast extract, was placed in a 90L jar fermenter. *Escherichia coli* ATCC11303 strain, which has been pre-cultured in 1L of a medium with the above composition, was inoculated in the fermenter and cultured at 37 °C under aeration and agitation. After 12 hours the culture was completed, and butter-like cells were collected by centrifugation.

18g of PAS-880 (Nitto Boseki Co., Ltd) adjusted to around pH 7 with alkaline and 27g of deionized water were mixed well for uniform dispersion of 40g of the above cells. 300 ml of ion exchange resin (Amberlight IRA-96SB, C1 type, manufactured by Organo Corp., 0.5mm of average grain diameter) and previously obtained cell suspension were added in a 6L Kjeldahl shortneck flask. This flask was dried under reduced pressure for 6 hours while rotating at 30 °C so that the cells were bound to the surface of the ion exchange resin, thereby obtaining an immobilized biocatalyst. The water content of this immobilized biocatalyst measured with a Carl-Fisher moisture meter was 6 wt%.

The immobilized biocatalyst prepared as described above was dipped in 20% ammonium fumarate solution at pH 8.5 at 4 °C overnight. Then 25ml of the resulting product was packed in a column with a jacket. Hot water at 30 °C was allowed to circulate in the jacket and the temperature of a reactor was set at 30 °C.

Substrate solution in a bottle with a cap had a composition per litter consisted of 200 g of fumaric acid, 200g of 25% aqueous ammonia, and 0.25g of magnesium sulfate heptahydrate, and adjusted to pH 8.3 with ammonia. The substrate solution was introduced into the column at a rate of 25ml per hour through a Teflon tube so that reaction took place continuously.

Analysis of the reaction solution 6 hours after that the reaction has started revealed that L-aspartic acid was generated as reaction products in equimolar quantity with the fumaric acid consumed. The reaction conversion rate was 99.7%. One month and four months after that the reaction has started, the conversion rate was kept at 99.7%.

103g of sodium hydroxide, 1.5-fold moles for ammonia, was added to 1L of 23% ammonium L-aspartate solution (specific gravity 1.11). Then the solution (specific gravity 1.21) was added in a 2L Kjeldahl shortneck flask, and concentrated under reduced pressure with an evaporator while dipping in a thermostat at 60 °C. About 60 minutes after this manipulation, 677g of 1.5-sodium L-aspartate solution remained in the Kjeldahl shortneck flask. No precipitated crystal was seen during and after the manipulation. The concentration of this solution was 42.01 W/W % when it was estimated in salts. That is, the solution could be concentrated about 1.8 fold.

No precipitated crystal was observed even when this concentrated solution was allowed to stand at 20 °C for 3 days. Moreover when refrigerated at 4 °C for 3 days, no precipitated crystal was observed.

### Example 16

103g of sodium hydroxide, 1.5-fold moles for ammonia, was added to 1L of 23% ammonium L-aspartate solution obtained in the same manner as described in Example 15. Then the solution was added in a 2L Kjeldahl shortneck flask, and concentrated under reduced pressure with an evaporator while dipping in a thermostat at 60 °C. About 80 minutes after this manipulation, 657g of 1.5-sodium L-aspartate solution remained in the Kjeldahl shortneck flask. No precipitated crystal was seen during and after the manipulation. The concentration of this solution was 43.31 w/w% when it was estimated in salts. That is, the solution could be concentrated about 1.9 fold.

No precipitated crystal was observed even when this concentrated solution was allowed to stand at 20 °C for 3 days. Moreover when refrigerated at 4 °C for 3 days, no precipitated crystal was observed.

### Example 17

103g of sodium hydroxide, 1.5-fold moles for ammonia, was added to 1L of 23% ammonium L-aspartate solution obtained in the same manner as described in Example 15. Then the solution was added in a 2L Kjeldahl shortneck flask, and concentrated under reduced pressure with an evaporator while dipping in a thermostat at 60 °C. About 90 minutes after this manipulation, 624g of 1.5-sodium L-aspartate solution remained in the Kjeldahl shortneck flask. No precipitated crystal was seen during and after the manipulation. The concentration of this solution was 45.58 w/w% when it was estimated in salts. That is, the solution could be concentrated about 2 fold. The viscosity of this concentrated solution measured were 0.013 Pas·s at 40 °C, 0.021 Pas·s at 25 °C, and 0.055 Pas·s at 4 °C. No precipitated crystal was observed even when this concentrated solution was allowed to stand at 20 °C for 3 days. Moreover, when refrigerated at 4 °C for 3 days, no precipitated crystal was observed.

### Example 18

103g of sodium hydroxide, 1.5-fold moles for ammonia, was added to 1L of 23% ammonium L-aspartate solution obtained in the same manner as described in Example 15. Then the solution was added in a 2L Kjeldahl shortneck flask, and concentrated under reduced pressure with an evaporator while dipping in a thermostat at 60 °C. About 90 minutes after this manipulation, 618g of 1.5-sodium L-aspartate solution remained in the Kjeldahl shortneck flask. No precipitated crystal was seen during and after the manipulation. The concentration of this solution was 46 w/w% when it was estimated in salts. That is, the solution could be concentrated about 2 fold. The specific gravity of this concentrated solution was 1.32.

No precipitated crystal was observed even when this concentrated solution was allowed to stand at 20 °C for 3 days. When refrigerated at 4 °C, however, precipitated crystal was observed.

### Example 19

103g of sodium hydroxide, 1.5-fold moles for ammonia, was added to 1L of 23% ammonium L-aspartate solution obtained in the same manner as described in Example 15. Then the solution was added in a 2L Kjeldahl shortneck flask, and concentrated under reduced pressure with an evaporator while dipping in a thermostat at 60 °C. About 100 minutes after this manipulation, 592g of 1.5-sodium L-aspartate solution remained in the Kjeldahl shortneck flask. No precipitated crystal was seen during and after the manipulation. The concentration of this solution was 48.05 w/w% when it was estimated in salts. That is, the solution could be concentrated about 2.1 fold. When this solution was allowed to stand at 20 °C, however, precipitated crystal was observed.

### Example 20

103g of sodium hydroxide, 1.5 fold moles for ammonia, was added to 1L of 23% ammonium L-aspartate solution obtained in the same manner as described in Example 15. Then the solution was added in a 2L Kjeldahl shortneck flask, and concentrated under reduced pressure with an evaporator while dipping in a thermostat at 60 °C. About 100 minutes after this manipulation, 584g of 1.5-sodium L-aspartate solution remained in the Kjeldahl shortneck flask. The concentration of this solution was 49 w/w% when it was estimated in salts. No precipitated crystal was observed during the manipulation at 60 °C. When the solution was transferred to another container, however, precipitated crystal was observed.

### Example 21

103g of sodium hydroxide, 1.5-fold moles for ammonia, was added to 1L of 23% ammonium L-aspartate solution obtained in the same manner as described in Example 15. Then the solution was added in a 2L Kjeldahl shortneck flask, and concentrated under reduced pressure with an evaporator while dipping in a thermostat at 60 °C. The behavior (dissolution, crystallization, or freezing) of 1.5-sodium L-aspartate at each temperature and concentration was shown in Table 3.

**Table 3**

| Behavior of 1.5-sodium L-aspartate solution at each temperature and concentration | | | | | | | | | |
|---|---|---|---|---|---|---|---|---|---|
| Storage temp. (°C) | Concentration of 1.5-sodium L-aspartate (w/w%) | | | | | | | | |
| | 21 | 25 | 28 | 32 | 42 | 43 | 46 | 48 | 49 |
| 60 | ○ | ○ | ○ | ○ | ○ | ○ | ○ | ○ | ○ |
| 40 | ○ | ○ | ○ | ○ | ○ | ○ | ○ | ○ | precipitated |
| 30 | ○ | ○ | ○ | ○ | ○ | ○ | ○ | ○ | precipitated |
| 25 | ○ | ○ | ○ | ○ | ○ | ○ | ○ | precipitated | precipitated |
| 10 | ○ | ○ | ○ | ○ | ○ | ○ | precipitated | precipitated | precipitated |
| 4 | ○ | ○ | ○ | ○ | ○ | ○ | precipitated | precipitated | precipitated |
| 0 | ○ | ○ | ○ | ○ | ○ | ○ | precipitated | precipitated | precipitated |
| -5 | ○ | ○ | ○ | ○ | ○ | ○ | precipitated | precipitated | precipitated |
| -15 | ○ | ○ | ○ | ○ | ○ | ○ | precipitated | precipitated | precipitated |
| -20 | frozen | frozen | *frozen | ○ | ○ | ○ | precipitated | precipitated | precipitated |
| -25 | frozen | frozen | frozen | frozen | *frozen | ○ | precipitated | precipitated | precipitated |
| ○ : dissolved, *frozen: partially frozen | | | | | | | | | |

### Example 22

60 L of a medium at pH 6.3 with a composition per litter of distilled water consisted of 10 g of fumaric acid, 5g of ammonium sulfate, 1g of monopotassium phosphate, 3g of dipotassium phosphate, 0.5g of magnesium sulfate heptahydrate, 5.5g of NaOH and 20g of yeast extract, was placed in a 90L jar fermenter. *Escherichia coli* ATCC11303 strain, which has been pre-cultured in 1L of a medium with the above composition, was inoculated in the fermenter and cultured at 37 °C under aeration and agitation. After 12 hours the culture was completed, and butter-like cells were collected by centrifugation.

18g of PAS-880 (Nitto Boseki Co., Ltd) adjusted to around pH 7 with alkaline and 27g of deionized water were mixed well for uniform dispersion of 40g of the above cells. 300 ml of ion exchange resin (Amberlight IRA-96SB, C1 type, manufactured by Organo Corp., 0.5mm of average grain diameter) and previously obtained cell suspension were added in a 6L Kjeldahl shortneck flask. This flask was dried under reduced pressure for 6 hours while rotating at 30 °C so that the cells were bound to the surface of the ion exchange resin, thereby obtaining an immobilized biocatalyst. The water content of this immobilized biocatalyst measured with a Carl-Fisher moisture meter was 6 wt%.

The immobilized biocatalyst prepared as described above was dipped in 20% ammonium fumarate solution at pH 8.5 at 4 °C overnight. Then 25ml of the resulting product was packed in a column with a jacket. Hot water at 30 °C was allowed to circulate in the jacket and the temperature of a reactor was set at 30 °C.

Substrate solution in a bottle with a cap had a composition per litter consisted of 200 g of fumaric acid, 200g of 25% aqueous ammonia, and 0.25g of magnesium sulfate heptahydrate, and adjusted to pH 8.3 with ammonia. The substrate solution was introduced into the column at a rate of 25ml per hour through a Teflon tube so that reaction took place continuously.

Analysis of the reaction solution 6 hours after that the reaction has started revealed that L-aspartic acid was generated as reaction products in equimolar quantity with the fumaric acid consumed. The reaction conversion rate was 99.7%. One month and four months after that the reaction has started, the conversion rate was kept at 99.7%.

64g of calcium hydroxide, 0.5-fold moles for ammonia, was added to 1L of 23% ammonium L-aspartate solution (specific gravity 1.11). Then the solution was added in a 2L Kjeldahl shortneck flask, and concentrated under reduced pressure with an evaporator while dipping in a thermostat at 60 °C. About 1 hour after this manipulation, 639g of 0.5-calcium L-aspartate solution remained in the Kjeldahl shortneck flask. No precipitated crystal was seen during and after the manipulation. The concentration of this solution was 40.99 W/W % when it was estimated in salts. That is, the solution could be concentrated about 1.8 fold. The specific gravity of this concentrated solution was 1.26.

No precipitated crystal was observed even when this concentrated solution was allowed to stand at 20 °C for 3 days. Moreover when refrigerated at 4 °C, no precipitated crystal was observed.

### Example 23

64g of calcium hydroxide, equimolar with ammonia, was added to 1L of 23% ammonium L-aspartate solution obtained in the same manner as described in Example 22. Then the solution was added in a 2L Kjeldahl shortneck flask, and concentrated under reduced pressure with an evaporator while dipping in a thermostat at 60 °C. About 80 minutes after this manipulation, 582g of 0.5-calcium L-aspartate solution remained in the Kjeldahl shortneck flask. No precipitated crystal was seen during and after the manipulation. The concentration of this solution was 45.02 w/w% when it was estimated in salts. That is, the solution could be concentrated about 1.9 fold. The specific gravity of this solution was 1.28.

No precipitated crystal was observed even when this concentrated solution was allowed to stand at 20 °C for 3 days. Moreover when refrigerated at 4 °C for 3days, no precipitated crystal was observed.

### Example 24

64g of calcium hydroxide, equimolar with ammonia, was added to 1L of 23% ammonium L-aspartate solution obtained in the same manner as described in Example 22. Then the solution was added in a 2L Kjeldahl shortneck flask, and concentrated under reduced pressure with an evaporator while dipping in a thermostat at 60 °C. About 90 minutes after this manipulation, 557g of 0.5-calcium L-aspartate solution remained in the Kjeldahl shortneck flask. No precipitated crystal was seen during and after the manipulation. The concentration of this solution was 47.04 w/w% when it was estimated in salts. That is, the solution could be concentrated about 1.9 fold. The specific gravity of this solution was 1.33.

No precipitated crystal was observed even when this concentrated solution was allowed to stand at 20 °C for 3 days. Moreover when refrigerated at 4 °C for 3days, no precipitated crystal was observed.

### Example 25

69g of calcium hydroxide, equimolar with ammonia, was added to 1L of 23% ammonium L-aspartate solution obtained in the same manner as described in Example 22. Then the solution was added in a 2L Kjeldahl shortneck flask, and concentrated under reduced pressure with an evaporator while dipping in a thermostat at 60 °C. About 90 minutes after this manipulation, 524g of 0.5-calcium L-aspartate solution remained in the Kjeldahl shortneck flask. No precipitated crystal was seen during and after the manipulation. The concentration of this solution was 50.0 w/w% when it was estimated in salts. That is, the solution could be concentrated about 1.92 fold. The specific gravity of this solution was 1.37.

No precipitated crystal was observed even when this concentrated solution was allowed to stand at 20 °C for 3 days. When refrigerated at 4 °C, however, precipitated crystal was observed.

### Example 26

69g of calcium hydroxide, equimolar with ammonia, was added to 1L of 23% ammonium L-aspartate solution obtained in the same manner as described in Example 22. Then the solution was added in a 2L Kjeldahl shortneck flask, and concentrated under reduced pressure with an evaporator while dipping in a thermostat at 60 'C. About 90 minutes after this manipulation, 484g of 0.5-calcium L-aspartate solution remained in the Kjeldahl shortneck flask. No precipitated crystal was seen during and after the manipulation. The concentration of this solution was 54.02 w/w% when it was estimated in salts. That is, the solution could be concentrated about 2.3 fold. The specific gravity of this solution was 1.4.

No precipitated crystal was observed even when this concentrated solution was allowed to stand at 20 °C for 3 days. When refrigerated at 4 °C, however, precipitated crystal was observed.

### Example 27

69g of calcium hydroxide, equimolar with ammonia, was added to 1L of 23% ammonium L-aspartate solution obtained in the same manner as described in Example 22. Then the solution was added in a 2L Kjeldahl shortneck flask, and concentrated under reduced pressure with an evaporator while dipping in a thermostat at 60 °C. The behavior (dissolution, crystallization, or freezing) of 0.5-calcium L-aspartate at each temperature and concentration was shown in Table 4.

**Table 4**

| Behavior of 0.5-calcium L-aspartate solution at each temperature and concentration | | | | | | | | | |
|---|---|---|---|---|---|---|---|---|---|
| Storage temp. (°C) | Concentration of 0.5-calcium L-aspartate (w/w%) | | | | | | | | |
| | 25.3 | 28.8 | 32.9 | 36.7 | 40.9 | 44.7 | 47.3 | 50.2 | 54 |
| 60 | ○ | ○ | ○ | ○ | ○ | ○ | ○ | ○ | ○ |
| 40 | ○ | ○ | ○ | ○ | ○ | ○ | ○ | ○ | ○ |
| 30 | ○ | ○ | ○ | ○ | ○ | ○ | ○ | ○ | ○ |
| 25 | ○ | ○ | ○ | ○ | ○ | ○ | ○ | ○ | ○ |
| 10 | ○ | ○ | ○ | ○ | ○ | ○ | ○ | ○ | ○ |
| 4 | ○ | ○ | ○ | ○ | ○ | ○ | ○ | ○ | ○ |
| 0 | ○ | ○ | ○ | ○ | ○ | ○ | ○ | ○ | ○ |
| -10 | frozen | frozen | frozen | frozen | frozen | frozen | ○ | ○ | ○ |
| -15 | frozen | frozen | frozen | frozen | frozen | frozen | frozen | ○ | ○ |
| -20 | frozen | frozen | frozen | frozen | frozen | frozen | frozen | frozen | frozen |
| -25 | frozen | frozen | frozen | frozen | frozen | frozen | frozen | frozen | frozen |
| ○ : dissolved | | | | | | | | | |

### Example 28

60 L of a medium at pH 6.3 with a composition per litter of distilled water consisted of 10 g of fumaric acid, 5g of ammonium sulfate, 1g of monopotassium phosphate, 3g of dipotassium phosphate, 0.5g of magnesium sulfate heptahydrate, 5.5g of NaOH and 20g of yeast extract, was placed in a 90L jar fermenter. *Escherichia coli* ATCC11303 strain, which has been pre-cultured in 1L of a medium with the above composition, was inoculated in the fermenter and cultured at 37 °C under aeration and agitation. After 12 hours the culture was completed, and butter-like cells were collected by centrifugation.

18g of PAS-880 (Nitto Boseki Co., Ltd) adjusted to around pH 7 with alkaline and 27g of deionized water were mixed well for uniform dispersion of 40g of the above cells. 300 ml of ion exchange resin (Amberlight IRA-96SB, C1 type, manufactured by Organo Corp., 0.5mm of average grain diameter) and previously obtained cell suspension were added in a 6L Kjeldahl shortneck flask. This flask was dried under reduced pressure for 6 hours while rotating at 30 °C so that the cells were bound to the surface of the ion exchange resin, thereby obtaining an immobilized biocatalyst. The water content of this immobilized biocatalyst measured with a Carl-Fisher moisture meter was 6 wt%.

The immobilized biocatalyst prepared as described above was dipped in 20% ammonium fumarate solution at pH 8.5 at 4 °C overnight. Then 25ml of the resulting product was packed in a column with a jacket. Hot water at 30 °C was allowed to circulate in the jacket and the temperature of a reactor was set at 30 °C.

Substrate solution in a bottle with a cap had a composition per litter consisted of 200 g of fumaric acid, 200g of 25% aqueous ammonia, and 0.25g of magnesium sulfate heptahydrate, and adjusted to pH 8.3 with ammonia. The substrate solution was introduced into the column at a rate of 25ml per hour through a Teflon tube so that reaction took place continuously.

Analysis of the reaction solution 6 hours after that the reaction has started revealed that L-aspartic acid was generated as reaction products in equimolar quantity with the fumaric acid consumed. The reaction conversion rate was 99.7%. One month and four months after that the reaction has started, the conversion rate was kept at 99.7%.

138g of sodium hydroxide, 2-fold moles for ammonia, was added to 1L of 23% ammonium L-aspartate solution (specific gravity 1.11). Then the solution (specific gravity 1.24) was added in a 2L Kjeldahl shortneck flask, and concentrated under reduced pressure with an evaporator while dipping in a thermostat at 60 °C. About 1 hour after this manipulation, 586g of 2-sodium L-aspartate solution remained in the Kjeldahl shortneck flask. No precipitated crystal was seen during and after the manipulation. The concentration of this solution was 51.77 W/W % when it was estimated in salts. That is, the solution could be concentrated about 2.3 fold. The specific gravity of this concentrated solution was 1.39. The viscosity of this solution measured were 0.043 Pas·s at 40 °C, 0.1 Pas·s at 25 °C, 0.8 Pas·s at 4 °C, and 1.88 Pas·s at -5 °C. The boiling point of this solution measured was 110 °C.

No precipitated crystal was observed even when this concentrated solution was allowed to stand at 20 °C for 3 days. Moreover when refrigerated at 4 °C, no precipitated crystal was observed. Subsequent refrigeration at -5 °C of this solution also resulted in no precipitated crystal.

### Example 29

138g of sodium hydroxide, 2-fold moles for ammonia, was added to 1L of 23% ammonium L-aspartate solution obtained in the same manner as described in Example 28. Then the solution was added in a 2L Kjeldahl shortneck flask, and concentrated under reduced pressure with an evaporator while dipping in a thermostat at 60 °C. About 80 minutes after this manipulation, 552g of 2-sodium L-aspartate solution remained in the Kjeldahl shortneck flask. No precipitated crystal was seen during and after the manipulation. The concentration of this solution was 54.95 w/w% when it was estimated in salts. That is, the solution could be concentrated about 2.4 fold. The specific gravity of this solution was 1.44.

No precipitated crystal was observed even when this concentrated solution was allowed to stand at 20 °C for 3 days. Moreover when refrigerated at 4 °C for 3days, no precipitated crystal was observed. Subsequent refrigeration at -5 °C of this solution also resulted in no precipitated crystal.

### Example 30

138g of sodium hydroxide, 2-fold moles for ammonia, was added to 1L of 23% ammonium L-aspartate solution obtained in the same manner as described in Example 28. Then the solution was added in a 2L Kjeldahl shortneck flask, and concentrated under reduced pressure with an evaporator while dipping in a thermostat at 60 °C. About 90 minutes after this manipulation, 518g of 2-sodium L-aspartate solution remained in the Kjeldahl shortneck flask. No precipitated crystal was seen during and after the manipulation. The concentration of this solution was 58.55 w/w% when it was estimated in salts. That is, the solution could be concentrated about 2.5 fold. The specific gravity of this solution was 1.51.

No precipitated crystal was observed even when this concentrated solution was allowed to stand at 20 °C for 3 days. Moreover when refrigerated at 4 °C for 3days, no precipitated crystal was observed. Subsequent refrigeration at -5 °C of this solution also resulted in no precipitated crystal.

### Example 31

138g of sodium hydroxide, 2-fold moles for ammonia, was added to 1L of 23% ammonium L-aspartate solution obtained in the same manner as described in Example 28. Then the solution was added in a 2L Kjeldahl shortneck flask, and concentrated under reduced pressure with an evaporator while dipping in a thermostat at 60 °C. About 90 minutes after this manipulation, 483g of 2-sodium L-aspartate solution remained in the Kjeldahl shortneck flask. No precipitated crystal was seen during and after the manipulation. The concentration of this solution was 62.8 w/w% when it was estimated in salts. That is, the solution could be concentrated about 2.7 fold. The specific gravity of this solution was 1.52. The viscosity of this solution measured were 0.37 Pas·s s at 40 °C, 1.66 Pas·s at 25 °C, 17.4 Pas·s (16.4 Pas·s) at 4 °C, and 280 Pas·s at -5 °C. The boiling point of this solution measured was 116 °C.

No precipitated crystal was observed even when this concentrated solution was allowed to stand at 20 °C for 3 days. Moreover when refrigerated at 4 °C for 3days, no precipitated crystal was observed. Subsequent refrigeration at -5 °C of this solution also resulted in no precipitated crystal.

### Example 32

138g of sodium hydroxide, 2-fold moles for ammonia, was added to 1L of 23% ammonium L-aspartate solution obtained in the same manner as described in Example 28. Then the solution was added in a 2L Kjeldahl shortneck flask, and concentrated under reduced pressure with an evaporator while dipping in a thermostat at 60 °C. About 100 minutes after this manipulation, 433g of 2-sodium L-aspartate solution remained in the Kjeldahl shortneck flask. No precipitated crystal was seen during and after the manipulation. The concentration of this solution was 70.05 w/w% when it was estimated in salts. That is, the solution could be concentrated about 3 fold. The specific gravity of this solution was 1.55. The viscosity of this solution measured were 1.39 Pas·s at 40 °C, 12.0 Pas·s (10.1 Pas·s) at 25 °C, 201 Pas·s (160 Pas·s) at 4 °C, and 9300 Pas s at -5 °C. The boiling point of this solution measured was 120 °C.

No precipitated crystal was observed even when this concentrated solution was allowed to stand at 20 °C for 3 days. Moreover when refrigerated at 4 °C for 3days, no precipitated crystal was observed. Subsequent refrigeration at -5 °C of this solution also resulted in no precipitated crystal.

### Example 33

138g of sodium hydroxide, 2-fold moles for ammonia, was added to 1L of 23% ammonium L-aspartate solution obtained in the same manner as described in Example 28. Then the solution was added in a 2L Kjeldahl shortneck flask, and concentrated under reduced pressure with an evaporator while dipping in a thermostat at 60 °C. About 100 minutes after this manipulation, 409g of 2-sodium L-aspartate solution remained in the Kjeldahl shortneck flask. No precipitated crystal was seen during and after the manipulation. The concentration of this solution was 74.2 w/w% when it was estimated in salts. That is, the solution could be concentrated about 3.2 fold. The specific gravity of this solution was 1.65. The viscosity of this solution measured were 25.4 Pas·s (18.9 Pas·s) at 40 °C, 212 Pas·s (212 Pas·s) at 25 °C. The viscosity of this solution was too high to measure at 4 °C and -5 °C. The boiling point of this solution measured was 127 °C.

No precipitated crystal was observed even when this concentrated solution was allowed to stand at 20 °C for 3 days. Moreover when refrigerated at 4 °C for 3days, no precipitated crystal was observed. Subsequent refrigeration at -5 °C of this solution also resulted in no precipitated crystal.

### Example 34

138g of sodium hydroxide, 2-fold moles for ammonia, was added to 1L of 23% ammonium L-aspartate solution obtained in the same manner as described in Example 28. Then the solution was added in a 2L Kjeldahl shortneck flask, and concentrated under reduced pressure with an evaporator while dipping in a thermostat at 60 °C. About 100 minutes after this manipulation, 371g of 2-sodium L-aspartate solution remained in the Kjeldahl shortneck flask. No precipitated crystal was seen during and after the manipulation. The concentration of this solution was 81.86 w/w% when it was estimated in salts. That is, the solution could be concentrated about 3.6 fold. The viscosity of this solution measured was 633 Pas·s (652 Pas·s) at 40 °C. The viscosity of this solution was too high to measure at 25 °C, 4 °C and -5 °C. The boiling point of this solution measured was 131 °C.

No precipitated crystal was observed even when this concentrated solution was allowed to stand at 20 °C for 3 days. Moreover when refrigerated at 4 °C for 3days, no precipitated crystal was observed. Subsequent refrigeration at -5 °C of this solution also resulted in no precipitated crystal.

### Example 35

138g of sodium hydroxide, 2-fold moles for ammonia, was added to 1L of 23% ammonium L-aspartate solution obtained in the same manner as described in Example 28. Then the solution was added in a 2L Kjeldahl shortneck flask, and concentrated under reduced pressure with an evaporator while dipping in a thermostat at 60 °C. About 100 minutes after this manipulation, 366g of 2-sodium L-aspartate solution remained in the Kjeldahl shortneck flask. No precipitated crystal was seen during and after the manipulation. The concentration of this solution was 82.9 w/w% when it was estimated in salts. That is, the solution could be concentrated about 3.6 fold. The viscosity of this solution was too high to measure even at 40 °C. The boiling point of this solution measured was 134 °C.

No precipitated crystal was observed even when this concentrated solution was allowed to stand at 20 °C for 3 days. Moreover when refrigerated at 4 °C for 3days, no precipitated crystal was observed. Subsequent refrigeration at -5 °C of this solution also resulted in no precipitated crystal.

### Example 36

1L of 23% 2-sodium L-aspartate solution obtained in the manner as described in Example 28 was added in a 2L Kjeldahl shortneck flask, and concentrated under reduced pressure with an evaporator while dipping in a thermostat at 60 °C. The behavior (dissolution, crystallization, or freezing) of 2-sodium L-aspartate solution at each temperature and concentration was shown in Table 5.

**Table 5**

| Behavior of 2-sodium L-aspartate solution at each temperature and concentration | | | | | | | | | |
|---|---|---|---|---|---|---|---|---|---|
| Storage temp. (°C) | Concentration of 2-sodium L-aspartate (w/w%) | | | | | | | | |
| | 21 | 25 | 28 | 31 | 51 | 62 | 70 | 74 | 82 |
| 60 | ○ | ○ | ○ | ○ | ○ | ○ | ○ | ○ | ○ |
| 40 | ○ | ○ | ○ | ○ | ○ | ○ | ○ | ○ | ○ |
| 30 | ○ | ○ | ○ | ○ | ○ | ○ | ○ | ○ | ○ |
| 25 | ○ | ○ | ○ | ○ | ○ | ○ | ○ | ○ | ○ |
| 10 | ○ | ○ | ○ | ○ | ○ | ○ | ○ | ○ | ○ |
| 4 | ○ | ○ | ○ | ○ | ○ | ○ | ○ | ○ | ○ |
| 0 | ○ | ○ | ○ | ○ | ○ | ○ | ○ | ○ | ○ |
| -5 | ○ | ○ | ○ | ○ | ○ | ○ | ○ | ○ | ○ |
| -15 | ○ | ○ | ○ | ○ | ○ | ○ | ○ | ○ | ○ |
| -20 | frozen | *frozen | ○ | ○ | ○ | ○ | ○ | ○ | ○ |
| -25 | frozen | frozen | *frozen | ○ | ○ | ○ | ○ | ○ | ○ |
| ○ : dissolved, *frozen: partially frozen | | | | | | | | | |

The present invention provides an aqueous solution containing L-aspartatic acid salt at a higher concentration at which no crystal is precipitated. This solution can maintain good storage and/or transport stability of L-aspartate over the long term.

All publications, patents and patent applications cited herein are incorporated herein by reference in their entirety.

## Claims

1. An ammonium L-aspartate solution containing ammonium L-aspartate whose concentration and temperature are maintained within the region bounded by A, B, C, D, E, F, G, H, I, and J as shown in Fig. 1.

2. A monosodium L-aspartate solution containing monosodium L-aspartate whose concentration and temperature are maintained within the region bounded by A, B, C, D, E, F, G, H, and I as shown in Fig. 2.

3. A 1.5-sodium L-aspartate solution containing 1.5-sodium L-aspartate whose concentration and temperature are maintained within the region bounded by A, B, C, D, E, F, G, H, I, J and K as shown in Fig. 3.

4. A 0.5-calcium L-aspartate solution containing 0.5-calcium L-aspartate whose concentration and temperature are maintained within the region bounded by A, B, C, D, E, F and G as shown in Fig. 4.

5. A 2-sodium L-aspartate solution containing 2-sodium L-aspartate whose concentration and temperature of aqueous solution of this salt are maintained within the region bounded by A, B, C, D, E, F and G as shown in Fig. 5.

6. A method for storing and/or transporting ammonium L-aspartate wherein ammonium L-aspartate is stored and/or transported in the ammonium L-aspartate solution of claim 1.

7. A method for storing and/or transporting monosodium L-aspartate wherein monosodium L-aspartate is stored and/or transported in the monosodium L-aspartate solution of claim 2.

8. A method for storing and/or transporting 1.5-sodium L-aspartate wherein 1.5-sodium L-aspartate is stored and/or transported in the 1.5-sodium L-aspartate solution of claim 3.

9. A method for storing and/or transporting 0.5-calcium L-aspartate wherein 0.5-calcium L-aspartate is stored and/or transported in the 0.5-calcium L-aspartate solution of claim 4.

10. A method for storing and/or transporting 2-sodium L-aspartate wherein 2-sodium L-aspartate is stored and/or transported in the 2-sodium L-aspartate solution of claim 5.
